# EUROPEAN PATENT APPLICATION

(11) **EP 4 309 698 A1**
(43) Date of publication of application: **24.01.2024**
(21) Application number: 22186460.6
(22) Date of filing: 22.07.2022
(51) Int. Cl.: A61M 5/142, A61M 1/06, F16H 13/06

(54) **PERISTALTIC PUMP WITH PLANETARY GEAR**

(71) Applicant: Medela Holding AG, 6340 Baar (CH)
(72) Inventor: VORAMWALD, René, 8604 Volketswil (CH)
(74) Representative: Grünecker Patent- und Rechtsanwälte PartG mbB

(57) **Abstract**

The present invention relates to a peristaltic pump (2) comprising planetary rollers (6) configured to revolve around a rotor (8) driving the planetary rollers (6), wherein the rotor (8) is configured to push the planetary rollers (6) against a flexible tube (10) such that the flexible tube (10) is squeezed between the planetary rollers (6) and a wall (14) of a pump housing (4). In order to facilitate the assembly and/or reduce the manufacturing cost and/or reduce the operating noise, a contact surface (20) between the rotor (8) and the planetary rollers (6) is toothless.

## Description

The present invention relates to a peristaltic pump. Peristaltic pumps are typically used to pump sterile fluids without exposing those fluids to contamination from exposed pump components. For example, a common application is pumping fluids through an infusion device. Peristaltic pumps are also used in heart-lung machines to circulate blood during bypass surgery, and in hemodialysis systems, since the peristaltic pump does not cause significant hemolysis, or rupture of the blood cells.

Peristaltic pumps are known, for example, from DE 690 06 239 T2, WO 2018/054833 A2, WO 2018/054834 A1, WO 2017/172217 A1, WO 2021/094352 A1 and EP 1 743 100 B1.

The documents WO 2017/172217 A1, WO 2018/054833 A2, WO 2018/054834 A1 and WO 2021/094352 A1, which belong to the applicant, refer to a peristaltic pump for supplying and/or suctioning a fluid to or from a human or animal body through a flexible tube, the peristaltic pump comprising a drive unit coupled to a sun wheel with a gear rim that meshes with gear rims of planetary wheels such that the planetary wheels revolve around the sun wheel and the flexible tube is squeezed between the planetary wheels and a wall of a pump housing.

The construction of such peristaltic pumps is complex and includes high-precision elements making the production of such peristaltic pumps expensive. Further, the operating noise that is created by the known peristaltic pumps can be annoying or unpleasant.

An object of the present invention is to provide a peristaltic pump for overcoming at least one of the aforementioned drawbacks. In particular, the present invention wants to provide a peristaltic pump that is easier to assemble and/or can be manufactured with fewer costs and/or creates less operating noise.

As a solution to the above object, the present invention proposes a peristaltic pump with the features of claim 1. The peristaltic pump comprises planetary rollers configured to revolve around a rotor driving the planetary rollers, wherein the rotor is configured to push the planetary rollers against a flexible tube such that the flexible tube is squeezed between the planetary rollers and a wall of a pump housing. As it is generally known, a fluid is pumped through the flexible tube by the planetary rollers squeezing the flexible tube when the planetary rollers roll on the flexible tube as they revolve around the rotor, wherein the portion of the flexible tube surrounding the planetary rollers preferably forms a ring segment.

The peristaltic pump is characterized in that a contact surface between the rotor and the planetary rollers is toothless. Accordingly, a frictional contribution to the transmission of torque from the rotor to the planetary rollers through the contact surface is enhanced. Friction at the contact surface may even be the predominant mechanism for transferring torque from the rotor to the planetary rollers. Thereby, the operating noise of the peristaltic pump can be reduced and the peristaltic pump can be assembled more easily, in comparison to a peristaltic pump having intermeshing gears. Further, the peristaltic pump can be manufactured at lower costs, because a manufacturing step of forming teeth into the contact surface is omitted. Preferably, the contact surface is cylindrical.

The rotor can comprise a sun roller, which preferably has an essentially smooth outer peripheral surface. Accordingly, the outer peripheral surface of the sun roller can be the toothless contact surface between the rotor and the planetary rollers. The outer peripheral surfaces of the planetary rollers can also be essentially smooth; or have teeth and/or notches. Alternatively, the outer peripheral surfaces of the planetary rollers can be essentially smooth and the outer peripheral surface of the sun roller may have teeth. The rotor can consist of the sun roller.

According to a preferred embodiment of the present invention, the contact surface is formed by an elastic material. The elastic material may form the outermost peripheral layer of the sun roller and/or the planetary rollers. Alternatively, the sun roller and/or the planetary rollers may be formed entirely or at least partially of the elastic material. According to this preferred embodiment, friction between the rotor and the planetary rollers is enhanced and compensation for tolerance differences is promoted.

According to another preferred embodiment of the present invention, the peristaltic pump further comprises a rotatable support having supporting arms that are arranged inside the planetary rollers. Said rotatable support can secure a substantially unchanging circumferential distance between the planetary rollers. That is, the support prevents that one of the planetary rollers falls behind or runs on ahead which could end in a collision and/or blockage of two planetary rollers. The supporting arms can be connected through a rigid base element. Further, the supporting arms can be equally spaced from each other. Preferably, the supporting arms are arranged eccentrically inside the planetary rollers. In particular, according to the preferred embodiment, the planetary rollers are free of an axle box arrangement, which further simplifies the manufacturing and reduces manufacturing costs.

According to another preferred embodiment of the present invention, the support is loosely connected to the rotor via the planetary rollers. Preferably, at least one of the planetary rollers, which are driven by the rotor, acts as a driver for the support through physical contact with the respective supporting arm provided inside the planetary roller. There can be a backlash between the supporting arms and an inner circumferential surface of the planetary rollers. In any case, the supporting arms allow the planetary rollers to turn.

According to another preferred embodiment of the present invention, the support comprises two parts that sandwich the planetary rollers inbetween, the two parts of the support preferably being releasably connected to each other. Preferably, the two parts of the support sandwich the planetary rollers in a direction transverse and/or orthogonal to a rolling direction of the planetary rollers. Thereby, the planetary rollers and the support can be joined as a sub-assembly to facilitate arranging the planetary rollers between the flexible tube and the rotor while keeping the desired distance between the planetary rollers. At least one of the two parts can comprise a latch configured to snap in place with a corresponding lug provided by the other part.

According to another preferred embodiment of the present invention, the support comprises two separable rings, at least one of the rings comprising axially projecting ring segments forming at least partially the supporting arms. Opposite end faces of the projecting ring segments in a circumferential direction of the rings are preferably convex, which can secure smooth turning of the planetary rollers, even though there is physical contact between the supporting arms and the planetary rollers. Preferably, the number of projecting ring segments of each ring is equal to the number of supporting arms, the supporting arms being formed by joining the projecting ring segments of the one ring and the projecting ring segments of the other ring. In particular, the projecting ring segments may be provided with a lug or a latch. In a circumferential direction, the projecting ring segments of one ring may be alternately provided with a lug or a latch. For example, if there are four supporting arms, two projecting ring segments of the rings can be provided with a lug and the other two rings segments can be provided with a latch, the lugs and the latches being provided alternately in a circumferential direction.

According to another preferred embodiment of the present invention, the peristaltic pump comprises a flexible sleeve provided between the rotor and the planetary rollers and having the contact surface. For example, the flexible sleeve can be a silicone ring. The flexible sleeve can be provided as an outer cover on the sun roller. Alternatively or additionally, the flexible sleeve can be provided as an outer cover on the planetary rollers. Preferably, the flexible sleeve has a Shore A hardness of between 40 and 100. In particular, the flexible sleeve can have a Shore A hardness of 60 or 80.

Another aspect of the present invention is directed towards a peristaltic pump comprising planetary rollers configured to revolve around a rotor driving the planetary rollers, wherein the rotor is configured to push the planetary rollers against a flexible tube such that the flexible tube is squeezed between the planetary rollers and a wall of a pump housing, the peristaltic pump further comprising a drive unit with a drive shaft, and a contactless coupling between the drive shaft and the rotor for transferring torque from the drive shaft to the rotor. Due to the contactless coupling, a static physical barrier can be provided to separate the fluid pumped through the flexible tube from a motor usually operating in air and providing the drive energy for the rotor. Contactless couplings can also ease maintenance, since they allow a greater off axis error between the rotor and the drive shaft coupled to the motor. Preferably, the contactless coupling is a magnetic coupling.

The peristaltic pump according to this aspect can have a toothless contact surface between the rotor and the planetary rollers and/or have the features of one or more of the above-mentioned preferred embodiments.

In an embodiment relating to this aspect, the peristaltic pump can comprise a first sensor element revolving around the rotor at the same velocity as the planetary rollers and a stationary second sensor element configured to detect the revolution of the first sensor element. For example, the first sensor element can be fixed to the support. The second sensor element can be fixed to a sealing wall, said sealing wall preferably being parallel to the rolling direction of the planetary rollers. Preferably, the sealing wall provides a static physical barrier between the pump housing and the motor. The first sensor element and the second sensor element can have substantially the same radial distance from an axis of rotation. Due to the contactless coupling, it can easily happen that the drive shaft generates torque that exceeds the maximum amount that the rotor can take up, such that the drive shaft rotates faster than the rotor. For example, if the contactless coupling is a magnetic coupling, the drive shaft may slip by skipping corresponding magnets of the magnetic coupling. That is, the drive shaft could overtake the rotor and/or the rotor could stop unnoticed, if the rotation of the drive shaft is monitored only. However, by using the first and second sensor elements, one can check whether the velocity of the planetary rollers matches the transmission ratio of the rotation of the drive shaft.

Preferably, the first sensor element is provided in a receptacle of a supporting arm. The first sensor element can comprise at least two magnets, each magnet being provided in a receptacle of a supporting arm. In particular, two magnets of the first sensor element can be provided at diametrical points of the support. The second sensor element can comprise a sensor for detecting a magnitude or a change of a magnetic field, e.g. a hall sensor.

Preferably, the rotor comprises a conical section and a cylindrical section, the cylindrical section having the contact surface, wherein the rotor is axially displaceable from a storage position, in which the conical section contacts the planetary rollers such that the rotor does not push the planetary rollers against the flexible tube and the flexible tube is not squeezed between the planetary rollers and the wall of the pump housing, into an operating position, in which the cylindrical section contacts the planetary rollers such that the planetary rollers are pushed against the flexible tube and the flexible tube is squeezed between the planetary rollers and the wall of the pump housing.

In the sense of the present application, the term "contact surface" refers to a contact surface in an operating position of the rotor.

The edge of the conical section next to the adjacent cylindrical section can have a larger diameter than the cylindrical section. This can facilitate the axial displacement of the rotor from the storage position into the operating position, since the planetary rollers do not contact the toothless contact surface until they slip off said edge, which reduces friction during the aforementioned axial displacement. Preferably, the conical section and the cylindrical section are adjacent.

In a preferred embodiment, the peristaltic pump can comprise an essentially disc-shaped pump housing adapted to accommodate the flexible tube, the planetary rollers, the support and the rotor, wherein the pump housing has an aperture, in which the rotor is axially displaceable from the storage position into the operating position, and wherein the pump housing is provided with an inlet port and an outlet port that are connectable inside the pump housing via the flexible tube. The front face and the rear face of the pump housing can have an essentially circular portion and an essentially three-cornered portion, the essentially three-cornered portion preferably having the inlet port and the outlet port, and the essentially circular portion preferably having the aperture. The pump housing can comprise a base plate providing the rear face and a top cover providing the front face, the base plate and the top cover being releasably connectable and having a circumferential wall between the front face and the rear face. The top cover and/or the base can provide the circumferential wall. The circumferential wall can correspond to the wall of the pump housing of claim 1. The circumferential wall can surround the flexible tube completely.

The pump housing can be held by a casing, the casing preferably being connected with a container having a reservoir for the fluid that is to be supplied to or suctioned from a human or animal body. The container can be arranged at a rear face of the pump housing, wherein the drive unit is preferably arranged at the front face of the pump housing. The sealing wall is preferably provided by the casing.

Further details and advantages of the present invention will be obtained from the following description of an embodiment and the accompanying drawings, in which:
- Figure 1: is a cross-sectional view including a perspective portion at the right side,
- Figure 2: is a perspective top view of the pump housing without top cover,
- Figure 3a: is a perspective side view of the pump housing showing the rotor in the storage position,
- Figure 3b: corresponds to Figure 3a without top cover,
- Figure 4a: is a perspective side view of the pump housing showing the rotor in the operating position,
- Figure 4b: corresponds to Figure 4a without top cover,
- Figure 5: is an exploded view of the support, the planetary rollers, and the first sensor element,
- Figure 6: is a longitudinal view, and
- Figure 7: shows components of the pump housing and in the pump housing according to detail D in Figure 6.

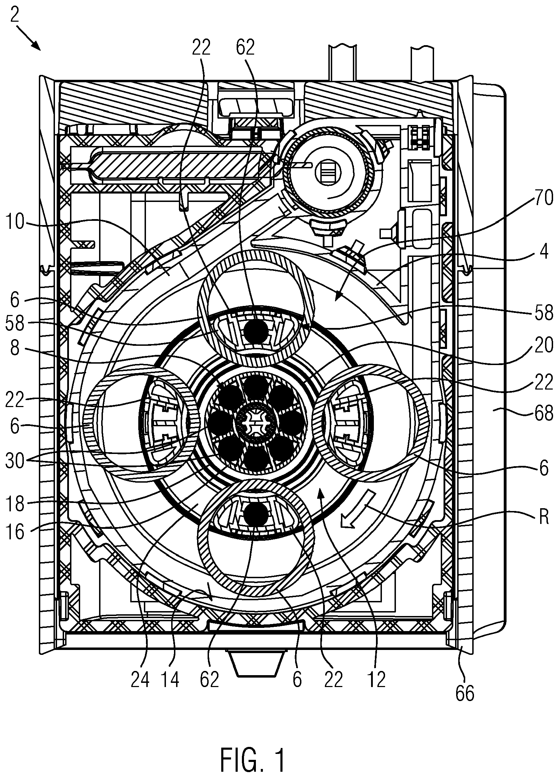

In Figure 1, an embodiment of a peristaltic pump according to the present invention is shown in a cross-sectional view. The peristaltic pump 2 comprises a pump housing 4 accommodating planetary rollers 6, a rotor 8, a flexible tube 10 and a support 12. In the embodiment, there are four planetary rollers 6 that are configured to revolve around the rotor 8 that drives the planetary rollers 6. The rotor 8 is configured to push the planetary rollers 6 against the flexible tube 10 such that the flexible tube 10 is squeezed between the planetary rollers 6 and a wall 14 of the pump housing 4. The rotor 8 comprises a sun roller 16 that carries a flexible sleeve 18. The flexible sleeve 18 has a toothless outer peripheral surface, which is in physical contact with the planetary rollers 6 to transmit torque onto the planetary rollers 6 and which forms a contact surface 20 between the planetary rollers 6 and the rotor 8.

The support 12 has four supporting arms 22, each supporting arm 22 being provided inside one planetary roller 6, and a rigid base 24 connecting the supporting arms 22, wherein the supporting arms 22 are equally spaced in a circumferential direction of the support 12, the circumferential direction of the support 12 corresponding to a rolling direction of the planetary rollers 6 indicated by an arrow with reference sign R.

The supporting arms 22 secure a substantially unchanging circumferential distance between the planetary rollers 6. That is, the support 12 prevents that one of the planetary rollers 6 falls behind or runs on ahead which could end in a collision and/or blockage of two planetary rollers 6. The supporting arms 22 are arranged eccentrically inside the planetary rollers 6 and opposing end faces 58 of the supporting arms 22 in a circumferential direction are convex. This allows the planetary rollers 6 to turn even though there is physical contact between the planetary rollers 6 and the support 12.

The support 12 is loosely connected to the rotor 8 via the planetary rollers 6. That is, at least one of the planetary rollers 6, which are driven by the rotor 8, acts as a driver for the support 12 through physical contact with the respective supporting arm 22 provided inside the planetary roller 6.

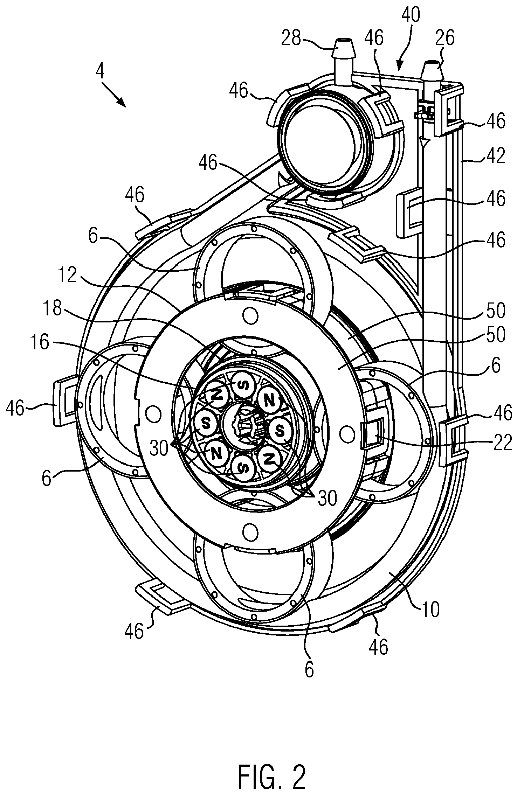

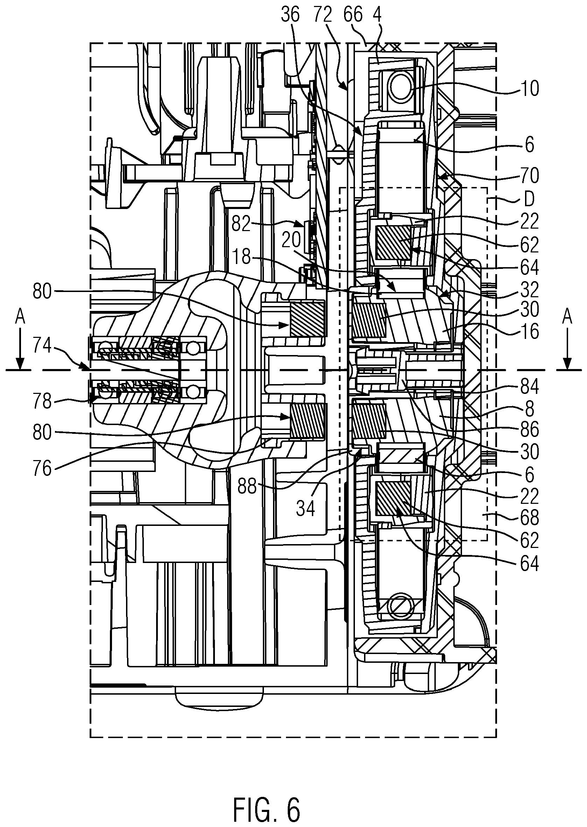

Figure 2 shows the pump housing 4 of the embodiment in a perspective top view. The pump housing 4 is provided with an inlet port 26 and an outlet port 28 that are connected inside the pump housing 4 via the flexible tube 10. The sun roller 16 accommodates rotor magnets 30 that are alternatingly oriented and belong to a contactless coupling that will explained later in connection with figure 6.

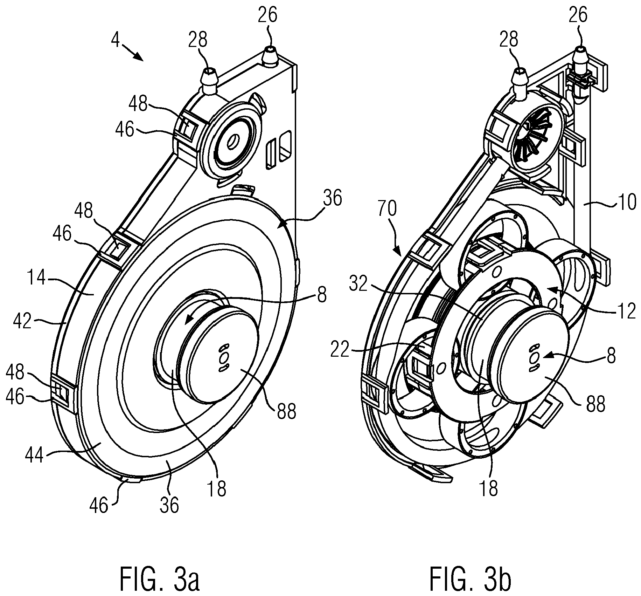

Figures 3a and 3b show the pump housing 4 of the embodiment in a perspective side view, wherein the rotor 8 is in a storage position. In the storage position, a portion of the sun roller 16 having a reduced diameter contacts the planetary rollers 6, such that the flexible tube 10 is not squeezed between the planetary rollers 6 and the wall 14 of the pump housing 4. This prolongs the lifetime of the flexible tube 10 before the peristaltic pump 2 is put into operation and/or when the peristaltic pump 2 is not used. The storage position will also allow sterilization of the flexible tube 10 prior to use of the peristaltic pump.

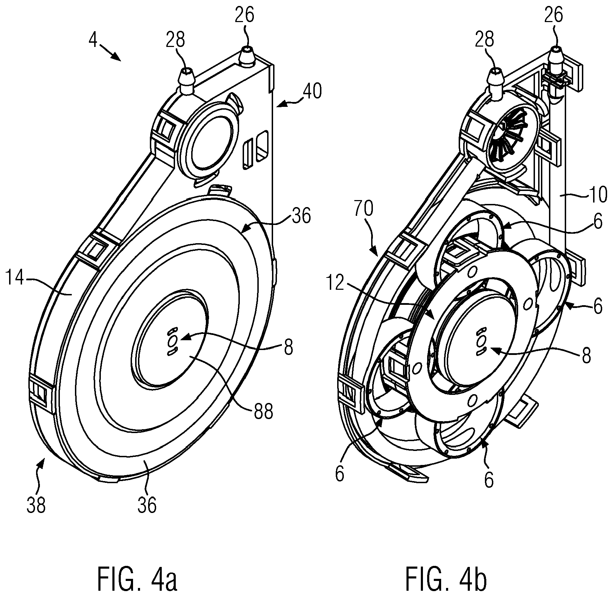

Figures 4a and 4b show the pump housing 4 of the embodiment in a perspective side view, wherein the rotor 8 is in an operating position. In the operating position, the contact surface 20 contacts the planetary rollers 6 such that the planetary rollers 6 are pushed against the flexible tube 10 and the flexible tube 10 is squeezed between the planetary rollers 6 and the wall 14 of the pump housing 4. The diameters of the wall 14, the planetary rollers 6 and the sun roller 16 are configured accordingly. The storage position of the rotor 8 is turned into the operating position by axial displacement of the rotor 8 towards the planetary rollers 6.

The pump housing 4 is essentially disc-shaped and has an aperture 34 at its front face 36, in which the rotor 8 is axially displaceable from the storage position into the operating position. The front face 36 and a rear face 70 of the pump housing 4 each have an essentially circular portion 38 and an essentially three-cornered portion 40 that are integrally connected, the essentially three-cornered portion 40 having the inlet port 26 and the outlet port 28.

The pump housing 4 has a base plate 42 providing the rear face 70 and a top cover 44 providing the front face 36, wherein the base plate 42 comprises outer lugs 46 and the top cover 44 comprises corresponding outer latches 48, such that the base plate 42 and the top cover 44 are releasably connectable.

A circumferential wall of the pump housing 4 between the front face 36 and the rear face is provided by the top cover 44 and surrounds the flexible tube 10. The circumferential wall corresponds to the wall 14 of the pump housing 4.

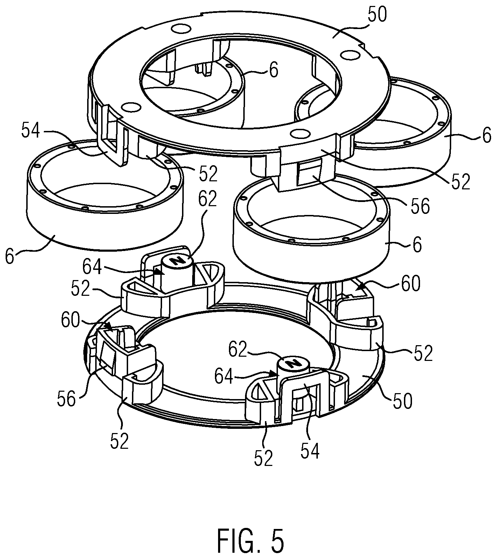

As shown in Figure 5, the support 12 (see Fig. 2) comprises two separable rings 50. Each ring 50 comprises four axially projecting ring segments 52, two inner lugs 54 and two inner latches 56, the projecting ring segments 52 being alternately provided with an inner lug 54 or an inner latch 56, wherein the inner lugs 54 of one ring 50 match with the inner lugs 56 of the other ring 50. The projecting ring segments 52 are equally spaced in the circumferential direction. By joining the rings 50, the projecting ring segments 52 form the supporting arms 22. Accordingly, opposite end faces 58 of the projecting ring segments 52 in a circumferential direction of the rings 50 are convex. One of the two rings 50 has two receptacles 60, the receptacles 60 being formed by two projecting ring segments 52 arranged diametrically and comprising the inner latches 56. Sensor magnets 62 are arranged in the receptacles 60 as a first sensor element 64.

Figure 6 shows the embodiment in a longitudinal view, wherein the left side in Figure 6 corresponds to a portion that is cut away in Fig. 1. The pump housing 4 is held by a casing 66 that is connected with a container 68 having a reservoir for the fluid that is to be supplied to or suctioned from a human or animal body. The container 68 is displayed in Fig.1 perspectively and arranged at the rear face 70 of the pump housing 4. A drive unit with a motor providing the drive energy for the rotor 8 is arranged at the front face 36 of the pump housing 4, the drive unit and the pump housing 4 being separated by a stationary sealing wall 72. The drive unit comprises a drive shaft 74 that carries a turret 76, the drive shaft 74 being rotateably supported via a rolling bearing 78.

The turret 76 is provided with drive magnets 80, which are arranged in alternating orientation in the circumferential direction and face the rotor magnets 30 at the other side of the sealing wall 72. When the drive magnets 80 turn, the rotor magnets 30 follow. That is, the drive magnets 80 and the rotor magnets 30 form a magnetic coupling, which is a contactless coupling.

A hall sensor is provided as a second sensor element 82, the second sensor element 82 being fixed to the sealing wall 72, wherein the second sensor element 82 and the sensor magnets 62 of the first sensor element 64 essentially have the same radial distance from an axis of rotation A. Thus, the second sensor element 82 is configured to detect the sensor magnets 62 when they pass at the other side of the sealing wall 72. The signals of the second sensor element 82 are evaluated to determine the velocity of the support 12, which corresponds to the rotational speed of the planetary rollers 6 around the rotor 8, in order to check whether said velocity matches the transmission ratio of the rotation of the drive shaft 74.

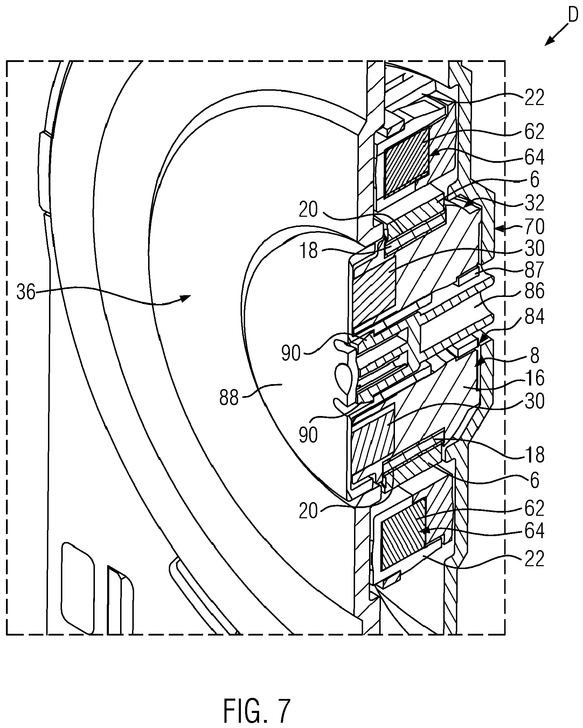

As shown in Figure 7, the sun roller 16 has a central hole 84 receiving - from one end - a pin 86, which is integrally connected with the base plate 42 of the pump housing 4, and - from the opposite end - a connector 87 of a cap 88, wherein the connector 87 forms a shaft of a bearing sleeve for the sun roller 16. The pin 86 comprises locking projections 90 that, when the sun roller 16 is pushed into the operating position, lock an axial position of the cap 88 and the sun roller 16. Further, the locking projections 90 prevent rotation of the connector 87. The cap 88 closes the aperture 34 when the rotor 8 is in the operating position.

### List of reference signs

- 2: peristaltic pump
- 4: pump housing
- 6: planetary roller
- 8: rotor
- 10: flexible tube
- 12: support
- 14: wall of the pump housing
- 16: sun roller
- 18: flexible sleeve
- 20: contact surface
- 22: supporting arm
- 24: rigid base
- 26: inlet port
- 28: outlet port
- 30: rotor magnet
- 32: conical section
- 34: aperture
- 36: front face
- 38: circular portion
- 40: three-cornered portion
- 42: base plate
- 44: top cover
- 46: outer lug
- 48: outer latch
- 50: ring
- 52: projecting ring segment
- 54: inner lug
- 56: inner latch
- 58: opposite end face
- 60: receptacle
- 62: sensor magnet
- 64: first sensor element
- 66: casing
- 68: container
- 70: rear face
- 72: sealing wall
- 74: drive shaft
- 76: turret
- 78: bearing
- 80: drive magnets
- 82: second sensor element
- 84: central hole
- 86: pin
- 87: connector
- 88: cap
- 90: locking projection
- A: axis of rotation
- R: rolling direction

## Claims

1. Peristaltic pump (2) comprising planetary rollers (6) configured to revolve around a rotor (8) driving the planetary rollers (6), wherein the rotor (8) is configured to push the planetary rollers (6) against a flexible tube (10) such that the flexible tube (10) is squeezed between the planetary rollers (6) and a wall (14) of a pump housing (4), **characterized in that** a contact surface (20) between the rotor (8) and the planetary rollers (6) is toothless.

2. Peristaltic pump (2) according to claim 1, wherein the contact surface (20) is formed by an elastic material.

3. Peristaltic pump (2) according to claim 1 or 2, further comprising a rotatable support (12) having supporting arms (22) that are arranged inside the planetary rollers (6).

4. Peristaltic pump (2) according to claim 3, wherein the supporting arms (22) are arranged eccentrically inside the planetary rollers (6).

5. Peristaltic pump (2) according to claim 3 or 4, wherein the support (12) is loosely connected to the rotor (8) via the planetary rollers (6).

6. Peristaltic pump (2) according to any one of claims 3 to 5, wherein the support (12) comprises two parts that sandwich the planetary rollers (6) inbetween, the two parts of the support (12) preferably being releasably connected to each other.

7. Peristaltic pump (2) according to any one of claims 3 to 6, wherein the support (12) comprises two separable rings (50), at least one of the rings (50) comprising axially projecting ring segments (52) forming at least partially the supporting arms (22), wherein opposite end faces (58) of the projecting ring segments (52) in a circumferential direction of the rings (50) are convex.

8. Peristaltic pump (2) according to any one of the preceding claims, further comprising a flexible sleeve (18) provided between the rotor (8) and the planetary rollers (6) and having the contact surface (20).

9. Peristaltic pump (2) according to claim 8, wherein the flexible sleeve (18) has a Shore A hardness of between 40 and 100, and preferably between 60 and 80.

10. Peristaltic pump (2) comprising planetary rollers (6) configured to revolve around a rotor (8) driving the planetary rollers (6), wherein the rotor (8) is configured to push the planetary rollers (6) against a flexible tube (10) such that the flexible tube (10) is squeezed between the planetary rollers (6) and a wall (14) of a pump housing (4), preferably according to any one of the preceding claims, further comprising a drive unit with a drive shaft (74), and a contactless coupling (30, 80) between the drive shaft (74) and the rotor (8) for transferring torque from the drive shaft (74) to the rotor (8).

11. Peristaltic pump (2) according to any one of the preceding claims, further comprising a first sensor element (64) revolving around the rotor (8) at the same speed as the planetary rollers (6), and a stationary second sensor element (82) configured to detect the revolution of the first sensor element (64).

12. Peristaltic pump (2) according to claim 11 depending on any one of claims 3 to 10, wherein the first sensor element (64) is provided in a receptacle (60) of a supporting arm (22).

13. Peristaltic pump (2) according to any one of the preceding claims, wherein the rotor (8) comprises a conical section (32) and a cylindrical section, the cylindrical section having the contact surface (20), and wherein the rotor (8) is axially displaceable from a storage position, in which the conical section (32) or a section with reduced diameter contacts the planetary rollers (6) such that the flexible tube (10) is not squeezed between the planetary rollers (6) and the wall (14) of the pump housing (4), into an operating position, in which the cylindrical section contacts the planetary rollers (6) such that the planetary rollers (6) are pushed against the flexible tube (10) and the flexible tube (10) is squeezed between the planetary rollers (6) and the wall (14) of the pump housing (4).

14. Peristaltic pump (2) according to claim 13, wherein an edge of the conical section (32) next to the cylindrical section has a larger diameter than the cylindrical section.

15. Peristaltic pump (2) according to claim 13 or 14, wherein the pump housing (4) has an aperture (34) in which the rotor (8) is axially displaceable from the storage position into the operating position, and wherein the pump housing (4) is provided with an inlet port (26) and an outlet port (28) that are connectable inside the pump housing (4) via the flexible tube (10).
